(19) Europäisches Patentamt
European Patent Office
Office européen des brevets

(11) Veröffentlichungsnummer: **0 165 572**
**A2**

(12) # EUROPÄISCHE PATENTANMELDUNG

(21) Anmeldenummer: 85107383.3

(22) Anmeldetag: 14.06.85

(51) Int. Cl.[4]: C 07 D 471/04
A 01 N 47/36
//(C07D471/04, 239:00, 221:00)

(30) Priorität: 20.06.84 DE 3422824

(43) Veröffentlichungstag der Anmeldung:
27.12.85 Patentblatt 85/52

(84) Benannte Vertragsstaaten:
AT BE CH DE FR GB IT LI LU NL SE

(71) Anmelder: CELAMERCK GmbH & Co. KG
Binger Strasse 173
D-6507 Ingelheim am Rhein(DE)

(72) Erfinder: Mengel, Rudolf, Dr. Dipl.-Chem.
Im Herzenacker 32
D-6535 Gau-Algesheim(DE)

(72) Erfinder: Pfleiderer, Wolfgang, Prof. Dr. Dr. hc
Universitätsstrasse 10
D-7750 Konstanz 1(DE)

(72) Erfinder: Linden, Gerbert, Dr. Dipl.-Landwirt
Turnierstrasse 44
D-6507 Ingelheim am Rhein(DE)

(72) Erfinder: Schneider, Gerhart, Dipl.-Biologe
Schleifmühlenweg 7a
D-6109 Mühltal 1(DE)

(54) **Neue herbizid wirksame Sulfonylharnstoffe.**

(57) Die Erfindung betrifft Verbindungen der Formel I

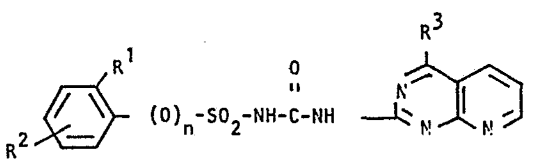

(I)

in der die Substituenten die im Text erläuterte Bedeutung haben, Verfahren zu deren Herstellung sowie deren Verwendung als herbizide und wachstumsregulierende Mittel sowie Verbindungen der Formal III

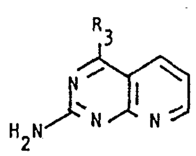

(III)

und Verfahren zu deren Herstellung.

EP 0 165 572 A2

Die Erfindung betrifft neue Pyrido [2,3-d] pyrimidin-2-yl-sulfonylharnstoffe, ihre Herstellung nach an sich bekannten Verfahren, die Verwendung der neuen Verbindungen bei der Bekämpfung unerwünschten Pflanzenwachstums sowie neue 2-Amino-pyrido [2,3-d]pyrimidine und deren Synthese.

Es wurde gefunden, daß die neuen Pyrido [2,3-d]pyrimidin-2-yl-harnstoffe der Formel

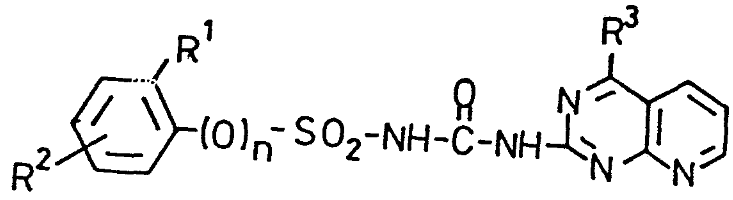

(I)

und ihre Salze mit Säuren und Basen gegen zahlreiche Unkräuter und Ungräser herbizid und bei einigen Kulturpflanzen wachstumsregulatorisch wirksam sind.

In der obigen Formel und im folgenden bedeutet

n 0 oder 1

$R^1$, $R^2$ die gleich oder verschieden sein können Wasserstoff, Halogen, Cyano, Nitro, Niederalkoxycarbonyl, gegebenenfalls halogensubstituiertes Niederalkyl, gegebenenfalls halogensubstituiertes Niederalkyloxy, gegebenenfalls halogensubstituiertes Niederalkylthio, gegebenenfalls halogensubstituiertes Niederalkenyl, gegebenenfalls halogensubstituiertes Niederalkenyloxy, gegebenenfalls halogensubstituiertes Niederalkenylthio, gegebenenfalls niederalkylsubstituierte Cycloalkyl, Di-(niederalkyl)amino, gegebenenfalls halogensubstituiertes Cyclopropylmethyl, gegebenenfalls halogensubstituiertes Cyclopropylmethyloxy, X $SO_2R^4$

$R^3$ Wasserstoff, gegebenenfalls halogensubstituiertes Niederalkyl, gegebenenfalls halogensubstituiertes Niederalkyloxy, gegebenenfalls halogensubstituiertes Niederalkylthio, Halogen, Amino, Hydroxy, Mercapto, Mono(niederalkyl)amino, Di(niederalkyl)amino.

$R^4$ Niederalkyl, gegebenenfalls ein- bis dreifach halogensubstituiertes Niederalkyl oder Niederalkyloxy, Cyclopropyl, Methyl, Cyclopropylmethoxy, Amino, Mono(niederalkyl)amino, Di(niederalkyl)amino

X Sauerstoff, NH oder N(Niederalkyl) oder direkte C-SBindung

bedeuten,
in freier Form und als Salze mit Säuren und Basen.

Hervorzuheben sindVerbindungen der Formel (I) in der

n 0,1

$R^1$ Wasserstoff, Nitro, Fluor, Chlor, Niederalkoxycarbonyl, gegebenenfalls halogensubstituiertes Niederalkyl, gegebenenfalls halogensubstituiertes Niederalkyloxy, gegebenenfalls halogensubstituiertes Niederalkylthio, gegebenenfalls halogensubstituiertes Niederalkenyl, gegebenenfalls halogensubstituiertes Niederalkenyloxy, gegebenenfalls halogensubstituiertes Niederalkenylthio,
gegebenenfalls halogensubstituiertes Cyclopropylmethyl,
gegebenenfalls halogensubstituiertes Cyclopropylmethyloxy,
Di-(niederalkyl)-amino, X $SO_2R^4$,

$R^2$ Wasserstoff, Chlor, Fluor

$R^3$ Wasserstoff, Fluor, Chlor, Niederalkyl, Niederalkyloxy, Niederalkylthio, Amino, Hydroxy, Mercapto, Amino, Mono-(niederalkyl)-amino, Di-(niederalkyl)-amino, Trifluormethyl

$R^4$ Niederalkyl, Niederalkyloxy, Cyclopropylmethoxy, Amino, Di-(niederalkyl)-amino,

X Sauerstoff, NH, N(Niederalkyl) oder direkte C-S-Bindung

bedeuten.

Bevorzugt sind Verbindungen der Formel (I) in der

n 0

$R^1$ Methoxycarbonyl, Chlor, Nitro

$R^2$ Wasserstoff

$R^3$ Amino, Methoxy, Dimethylamino, Methylthio, Chlor, Hydroxy, Mercapto, Methylamino

bedeuten.

Besonders hervorzuheben sind nachstehend genannte Verbindungen der Formel (I):

N-(4-Aminopyrido[2,3-d]pyrimidin-2-yl)-N'-(2-nitrophenylsulfonyl)-harnstoff

N-(2-Chlorphenylsulfonyl)-N'-(4-methoxy-pyrido[2,3-d]-pyrimidin-2-yl)harnstoff

N-(4-Aminopyrido[2,3-d]pyrimidin-2-yl)-N'-(2-methoxycarbonyl-phenylsulfonyl)harnstoff

N-(4-Dimethylaminopyrido[2,3-d]pyrimidin-2-yl)-N'-(2-methoxy-carbonylphenylsulfonyl)harnstoff

N-(2-Methoxycarbonylphenylsulfonyl)-N'-(4-methoxypyrido[2,3-d]-pyrimidin-2-yl)harnstoff

N-(4-Methoxypyrido[2,3-d]pyrimidin-2-yl-N'-(2-nitrophenylsulfonyl)-harnstoff

N-(2-Chlorphenylsulfonyl)-N'-(4-chlorpyrido[2,3-d]pyrimidin-2-yl)harnstoff

N-(2-Methoxycarbonylphenylsulfonyl)-N'-(4-methylthiopyrido-[2,3-d]pyrimidin-2-yl)harnstoff

Niederalkyl ist im Rahmen der obigen Definition bevorzugt eine geradkettige oder verzweigte Gruppe dieser Art mit bis zu 4 Kohlenstoffatomen, also Methyl, Ethyl, n-Propyl, Isopropyl, n-Butyl, sek-Butyl, iso-Butyl und tert-Butyl. Dies gilt auch, soweit der Niederalkylrest Bestandteil einer Alkoxy-, Alkylthio-, Alkoxycarbonyl-, oder Aminogruppe ist. Hervorzuheben sind Methyl, Methoxy, Methylthio, Methylamino und Dimethylamino.

Niederalkenylgruppen sind bevorzugt geradkettige oder verzweigte Gruppen mit 2 bis 6 Kohlenstoffatomen und ein oder zwei Doppelbindungen. Der Begriff umfaßt definitionsgemäß auch die verschiedenen Z- und E-Isomere. Hervorzuheben sind die Vinyl-, Allyl- und die isomeren Butenyl- und Pentenylgruppen. Dies gilt auch, soweit der Niederalkenylrest Bestandteil einer Niederalkenyloxy- oder Niederalkenylthiogruppe ist.

Unter Halogen sind Fluor, Chlor, Brom und Jod zu verstehen, vor allem Fluor, Chlor und in zweiter Linie Brom.

Mit halogensubstituierten Niederalkyl-, Niederalkyloxy- und Niederalkylthioresten sind Gruppen gemeint, deren Niederalkylteil wie zuvor definiert ist und mit 1 bis 6 Halogenatomen, die gleich oder verschieden sein können, substituiert ist.

Bevorzugt sind die Trifluormethyl-, Trichlormethyl-,Difluormethyl-, Dichlormethyl-, Fluormethyl-, Chlormethyl-, Brommethyl, 2,2,2-Trifluorethyl-, 2,2,2-Trichlorethyl, Monochlordifluormethyl- und Dichlormonofluormethylradikale.

Cycloalkylreste können gesättigte carbocyclische Ringsysteme der Ringgröße drei bis sieben sein. Bevorzugt ist das Cyclopropylsystem.

Mit halogensubstituierten Niederalkenyl-, Niederalkenyloxy- und Niederalkenylthioresten sind Gruppen gemeint, deren Niederalkenylteil wie zuvor definiert ist und mit 1 bis 6 Halogenatomen, die gleich oder verschieden sein können, substituiert ist.

Die neuen Verbindungen werden hergestellt, indem man
a) ein Phenyl- oder Phenoxysulfonylsiocyanat der Formel

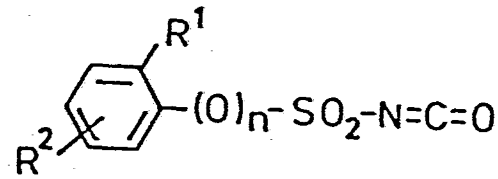

(II)

in der n, $R_1$ und $R_2$ die obige Bedeutung haben, mit einem Amin der Formel

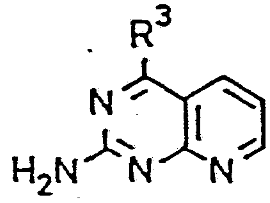

(III)

in der $R_3$ die obige Bedeutung hat umsetzt, oder daß man

b) ein Carbamat der Formel

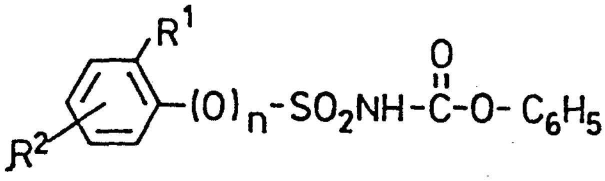

(IV)

in der n, $R_1$ und $R_2$ die obige Bedeutung haben, mit einem Amin der Formel (III) umsetzt, oder daß man

c) eine Verbindung der Formel

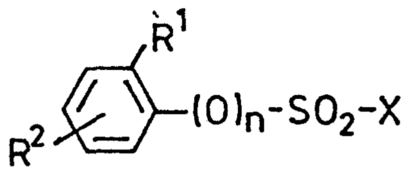

(V)

in der n, $R_1$ und $R_2$ die obige Bedeutung haben und X für Halogen, vorzugsweise Chlor steht, mit einem Imidokohlensäureester der Formel

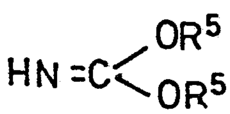

(VI)

in der $R_5$ für einen Niederalkyl-, Phenyl- oder Benzylrest steht zu einem Imidokohlensäureester der Formel

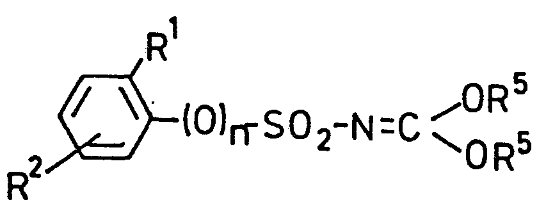

(VII)

umsetzt und danach mit einem Amin der Formel (III) umsetzt und den so erhaltenen Isoharnstoff der Formel

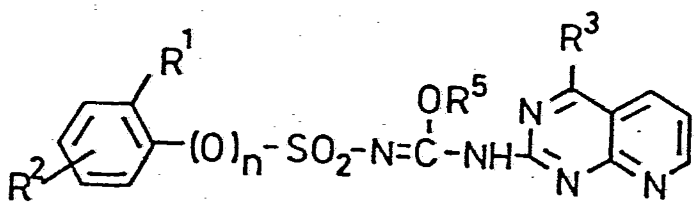

(VIII)

mit einer Halogenwasserstoffsäure zu einer Harnstoffverbindung der Formel (I) spaltet,

d) eine Verbindung der Formel

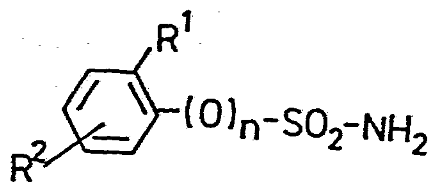

(IX)

in der n, $R^1$ und $R^2$ die obige Bedeutung haben mit einem Amin der Formel (III) und Carbodiimidazol nach dem in der deutschen Patentanmeldung Nr. 33 46 617 beschriebenen Verfahren umsetzt.

Gewünschtenfalls werden aus den nach a), b), c) oder d) erhaltenen Verbindungen der Formel I mit geeigneten Alkali- oder Erdalkaliverbindungen oder mit organischen Basen die entsprechenden Salze hergestellt. Dies geschieht vorzugsweise durch Umsetzung mit der berechneten Menge der Base und Isolierung des entstehenden Salzes.

Die Umsetzung gemäß a) erfolgt zweckmäßig in einem inerten aprotischen Lösungsmittel wie Methylenchlorid, Acetonitril. Die Umsetzung wird bei Raumtemperatur oder auch bei erhöhter Temperatur (bis zur Siedetemperatur des Reaktionsgemisches) zu Ende geführt, das Reaktionsprodukt isoliert und gewünschtenfalls nach üblichen Verfahren gereinigt. Wegen der z.T. sehr hohen Reaktivität insbesondere der Phenoxysulfonylisocyanate ist möglichst wasserfrei zu arbeiten.
Die als Ausgangsstoffe benötigten Phenoxysulfonylisocyanate der Formel II werden zweckmäßig nach dem von Lohaus, Chem. Ber. 105, 2791 - 2799 (1972), angegebenen Verfahren hergestellt. Im allgemeinen brauchen diese Verbindungen vor der Weiterverarbeitung nicht gereinigt zu werden.

Die Phenylsulfonylisocyanate der Formel II sind bekannt oder können analog bekannten Verbindungen dieses Typs hergestellt werden, z.B. nach DE-PS 817 602, US-PS 3 379 758, E-PS 21 641, DE-OS 32 28 101 oder Ulrich, Chem. Ber. 65, 369 (1965).

Die Carbamate der Formel IV können aus den entsprechenden Amiden der Formel

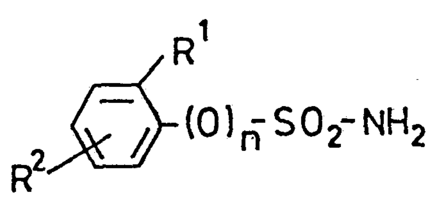

(IX)

und Diphenylcarbonat in Gegenwart einer Base in an sich bekannter Weise erhalten werden. Verbindungen der Formel (IX) sind nach üblichen Methoden herstellbar.

Phenoxysulfonylhalogenide bzw. Phenylsulfonylhalogenide der Formel (V) sind ebenso wie die Imidokohlensäureester (VI) seit langem bekannte Verbindungsklassen.

Die als Zwischenprodukte benötigten 2-Aminopyrido [2,3-d] pyrimidine der Formel (III) sind neu, bis auf die beiden Verbindungen mit $R^3$ = OH und $NH_2$.

Die Erfindung betrifft somit auch neue 2-Aminopyrido [2,3-d] pyrimidine der Formel (III) in denen

$R^3$ Wasserstoff gegebenenfalls halogensubstituiertes Niederalkyl, gegebenenfalls halogensubstituiertes Niederalkyloxy, gegebenenfalls halogensubstituiertes Niederalkylthio, Halogen, Mercapto, Mono(niederalkyl)amino, Di-(niederalkyl)amino bedeutet.

Bevorzugt sind Verbindungen der Formel (III), in der $R^3$ Wasserstoff,
Fluor, Chlor, Niederalkyl, Niederalkyloxy, Niederalkylthio, Mercapto, Mono(niederalkyl)amino und Di-(niederalkylamino) bedeutet.

Hervorzuheben sind die Verbindungen der Formel (III), in der $R^3$ für Methoxy, Dimethylamino, Methylthio, Chlor oder Mercapto steht.

Sie können nach einem literaturbekannten Verfahren (Bernetti et al; J. Org. Chem.; 27 (1962)2863) aus den leicht zugänglichen Diaminopyrimidinen der Formel

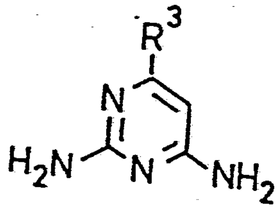

(X)

Mit Malondialdehyd, bzw. Derivaten des Malondialdehyds dargestellt werden.

Verbindungen der Formel (III), in denen $R^3$ für Halogen steht, können durch allgemein bekannte Halogenierungsreaktionen aus 2-Aminopyrido [2,3-d] pyrimidin-4-ol bzw. 4-thiol hergestellt werden oder sind durch die vorstehend beschriebene Cyclisierungsreaktion ausgehend von 2,6-Diamino-4-chlorpyrimidin und 1,1,3,3,-Tetramethoxypropan herstellbar.

Des weiteren können Verbindungen der Formel (III), in denen $R^3$ für Mercapto oder Alkylthio steht aus der 4-Hydroxyverbindung nach an sich bekannten Methoden mit Phosphorpentasulfid und gewünschtenfalls anschließender Alkylierung hergestellt werden.

4-Alkyl- bzw. Dialkylaminoverbindungen der Formel (III) können z.B. durch Substitutionsreaktionen ausgehend von dem 4-Halogen- bzw. 4-Methylthioderivat nach an sich bekannten Verfahren hergestellt werden.

Die Verbindungen der Formel I sind herbizid wirksam. Sie können vor und nach dem Auflaufen gegen zahlreiche Unkräuter und Ungräser eingesetzt werden, z.B. gegen Monocotyle: Granineen, wie Echinochloa crus-galli, Alopecurus myosuroides, Arena fatua, Setaria viridis, Digitaria sanguinalis oder Cyperaceen, wie Cyperus esculentus oder Dicotyle: wie Solanum nigrum, Sinapsis alba, Larium amplexicaule, Centaurea cyanus, Stellaria media, Veronica persicaria Galium aparine, Matricaria indora und andere.

Die gute Selektivität der neuen Verbindungen ermöglicht es, die Unkraut- bzw. Ungrasbekämpfung in zahlreichen Kulturen durchzuführen, beispielsweise in Weizen, Mais, Reis, Gerste, Kartoffeln, Tomaten, Sonnenblumen, Erbsen, Bohnen, Rüben, Baumwolle oder Soja.

In Abhängigkeit von der Aufwandmenge zeigen die Verbindungen der Formel I zudem wachstumsregulatorische Wirkung an wichtigen Kulturpflanzen, wie z.B. an Weizen, Gerste und Reis indem sie den Sproßaufbau dieser Pflanzen in erwünschtem vorteilhaften Sinne verändern, ohne die Kulturpflanze zu schädigen.

Die Wirkung besteht hauptsächlich in einer Reduzierung des Längenwachstums der Sproßinternodien, in einer "Stauchung" der Sproßachse. Es resultieren weniger hohe, aber standfestere Pflanzen, welche im Feld weniger leicht lagern und daher ohne Ertragsverluste durch Lagern maschinell beerntbar bleiben.

Für die Anwendung werden die Verbindungen der Formel I in an sich bekannter Weise mit üblichen Hilfs- und oder Trägerstoffen zu gebräuchlichen Formulierungen verarbeitet, z.B. zu Emulsionskonzentraten oder Suspensionspulvern, bei denen der Wirkstoffgehalt zwischen 10 und 95 Gewichtsprozent liegt und die für die Ausbringung mit Wasser bis zur gewünschten Wirkstoffkonzentration verdünnt werden. Jedoch können auch unverdünnt anwendbare Präparate hergestellt werden, etwa Granulate und Stäube. Hier liegt der Wirkstoffgehalt zwischen 0,1 und 10 Gewichtsprozent, vorzugsweise zwischen 0,3 und 3 Gewichtsprozent.
Die Aufwandmenge ist abhängig von einer Anwendungsart und kann in weiten Grenzen variieren. Sie liegt etwa zwischen 1,0 bis 1000 g/ha, vorzugsweise zwischen 10 und 500 g/ha bei herbizider Wirkung. Bei wachstumsregulatorischer Wirkung ist die Aufwandmenge in der Regel geringer.

Formulierungbeispiele
(Angabe der Zusammensetzung in Gewichtsprozent)

1) Stäubemittel

0,3 % einer Verbindung der Formel I
1,0 % Methylcellulose
98,7 % Talkum

2) Suspensionspulver

25 % einer Verbindung der Formel I
55 % Kaolin
10 % Kolloidale Kieselsäure
9 % Calciumligninsulfonat
1 % Natriumtetrapropylenbenzolsulfonat

3) Suspensionspulver

95 % einer Verbindung der Formel I
4 % Calciumligninsulfonat
1 % Natriumtetrapropylenbenzolsulfonat

4) Emulsionskonzentrat

10 % einer Verbindung der Formel I
80 % Dimethylformamid
6,5 % Tensiofix AS (Emulgator)
3,5 % Tensiofix DS (Emulgator)

5) Suspensionskonzentrat

20 % einer Verbindung der Formel I
3 % Dispergiermittel z.B. Naphthalinsulfat-Formaldehyd-Copolymer-Na-Salz
1 % Bentone EW (Mondmorillomit)
0,2 % Entschäumer (Silicon)
0,05 % Konservierungsmittel
ad 100 Wasser

Aus den Konzentraten 2. und 4. werden durch Vermischen mit Wasser Spritzbrühen hergestellt, die im allgemeinen zwischen 0,05 und 0,5 % Wirkstoff enthalten.

Die Herstellung der Verbindungen der Formel I sowie der Ausgangsstoffe wird im folgenden näher erläutert:

Beispiel 1

N-(4-Amino-pyrido/2,3-d/pyrimidin-2-yl'-N'-(2-nitrophenylsulfonyl) harnstoff

1,61 g 2,4-Diaminopyrido [2,3-d] pyrimidin, 2,5 g 2-Nitrophenylsulfonylisocyanat, 100 mg 1,5-Diazabicyclo-(5,4,0)-undec-5-en werden unter Feuchtigkeitsausschluß 4 Stunden in 40 ml Acetonitril zum Sieden erhitzt. Der Rückstand wird abgesaugt und in 40 ml siedenden Eisessig aufgenommen und heiß filtriert.
Der schwer lösliche Rückstand wird mit heißem Aceton gewaschen und getrocknet.

Ausbeute: 2,40 g (62 %) N-(4-Amino-pyrido [2,3] -pyrimidin-2-yl)-N'-(2-nitrophenylsulfonyl)harnstoff als farblose Kristalle vom Schmp. 240°C.

| | | | | |
|---|---|---|---|---|
| Elementaranalyse: | ber | C 43,19 | H 2,85 | N 25,18 |
| | gef. | C 43,40 | H 3,09 | N 24,98 |

Beispiel 2

N-(2-Chlorphenylsulfonyl)-N'-(4-methoxypyrido [2,3-d] -pyrimidin-2-yl)harnstoff.

1,76 g 2-Amino-4-methoxypyrido [2,3-d] pyrimidin werden unter Rühren und Feuchtigkeitsausschluß portionsweise in eine Lösung von 2,3 g 2-Chlorphenylsulfonylisocyanat in 20 ml Acetonitril eingetragen. Das Reaktionsgemisch erwärmt sich leicht. Es wird noch etwa 1 h nachgerührt. Nach Zugabe von Benzin wird der Niederschlag abfiltriert, mit Methanol aufgekocht und heiß abgesaugt.

Ausbeute: 3,6 g (91 %) N-(2-Chlorphenylsulfonyl)-N'-(4-methoxy-pyrido [2,3-d] pyrimidin-2-yl)harnstoff als farblose Kristalle vom Schmp. 195°C.

| | | | | |
|---|---|---|---|---|
| Elementaranalyse: | ber | C 45,75 % | H 3,07 % | N 17,7 % |
| | gef | C 45,74 % | H 3,09 % | N 18,16 % |

Entsprechend den Beispielen können auch die Verbindungen der Formel I erhalten werden, die in der folgenden Tabelle aufgeführt sind:

Tabelle 1:

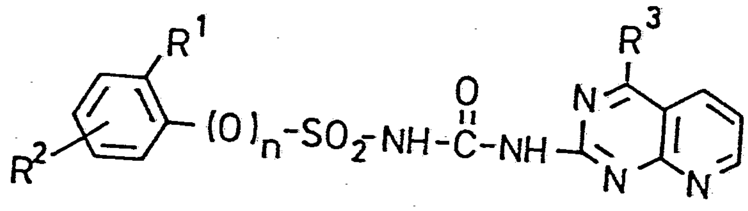

(I)

| Verb. No. | n | $R^1$ | $R^2$ | $R^3$ | physikal. Daten |
|---|---|---|---|---|---|
| 3 | 0 | $COOCH_3$ | H | $NH_2$ | Fp. 220°C |
| 4 | 0 | $COOCH_3$ | H | $N(CH_3)_2$ | Fp. 182°C |
| 5 | 0 | $COOCH_3$ | H | $N(CH_2CH_3)_2$ | |
| 6 | 0 | $COOCH_3$ | H | $OCH_3$ | Fp. 192°C |
| 7 | 1 | $COOCH_3$ | H | $NH_2$ | |
| 8 | 1 | $COOCH_3$ | H | $N(CH_3)_2$ | |
| 9 | 1 | $COOCH_3$ | H | $N(CH_2CH_3)_2$ | |
| 10 | 1 | $COOCH_3$ | H | $O-CH_3$ | |
| 11 | 0 | $COOCH_3$ | 4 Cl | $S-CH_3$ | |
| 12 | 1 | $COOC_2H_5$ | 4 $CH_3$ | $O-CH_3$ | |
| 13 | 0 | $COOC_4H_9$ | 4 F | Cl | |
| 14 | 0 | Cl | H | $NH_2$ | Fp. 240°C |
| 15 | 0 | Cl | H | $N(CH_3)_2$ | |
| 16 | 0 | Cl | H | $CH(CH_3)_2$ | |
| 17 | 0 | Cl | H | $S-CH_3$ | |
| 18 | 0 | Cl | 4 $CH_3$ | Cl | |
| 19 | 0 | F | H | $CH(CH_3)_2$ | |
| 20 | 1 | Cl | H | $NH_2$ | |

| Verb. No. | n | $R^1$ | $R^2$ | $R^3$ | physikal. Daten |
|---|---|---|---|---|---|
| 21 | 1 | Cl | H | $N(CH_3)_2$ | |
| 22 | 0 | $CH_2$-CH=$CH_2$ | H | $NH_2$ | |
| 23 | 0 | $CH_2$-CH=$CH_2$ | H | $OCH_3$ | |
| 24 | 1 | $CH_2$-CH=$CH_2$ | 4 $CH_3$ | Cl | |
| 25 | 1 | $CH_2$-CH=$CH_2$ | 4 Cl | S-$CH_3$ | |
| 26 | 0 | $CF_3$ | H | $NH_2$ | |
| 27 | 1 | $CF_3$ | H | $OCH_3$ | |
| 28 | 0 | $CH_2CCl_3$ | H | $NH_2$ | |
| 29 | 0 | $CH_2CCl_3$ | H | $OCH_3$ | |
| 30 | 0 | $C_4H_9$ | H | NH-$CH_3$ | |
| 31 | 0 | $CH_3O$ | 4 $CH_3$ | O-$CH_3$ | |
| 32 | 0 | $CH_3O$ | H | F | |
| 33 | 1 | $CH_3O$ | H | F | |
| 34 | 0 | $CH_3S$ | H | O-$CH_3$ | |
| 35 | 0 | $CF_3O$ | H | $NH_2$ | |
| 36 | 0 | $CF_3O$ | H | $N(CH_3)_2$ | |
| 37 | 0 | $CF_3O$ | H | O-$CH_3$ | |
| 38 | 0 | $CH_2$-CH=CH-$CH_3$ | H | O-$CH_3$ | |
| 39 | 1 | $CH_2$-CH=CH-$CH_3$ | H | $NH_2$ | |
| 40 | 0 | $CH_2$-CH=CH-$CH_3$ | H | $NH_2$ | |
| 41 | 0 | $N(CH_3)_2$ | H | O-$CH_3$ | |
| 42 | 0 | $CH_2$-C 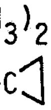 | H | O-$CH_3$ | |
| 43 | 0 | $SO_2CH_3$ | H | $NH_2$ | |
| 44 | 0 | $SO_2OCH_3$ | H | O-$CH_3$ | |
| 45 | 0 | $NHSO_2CH_3$ | H | O-$CH_3$ | |

| Verb. No. | n | $R^1$ | $R^2$ | $R^3$ | physikal. Daten |
|---|---|---|---|---|---|
| 46 | 1 | $NHSO_2CH_3$ | H | $O-CH_3$ | |
| 47 | 0 | $N(CH_3)SO_2CH_3$ | H | $NH_2$ | |
| 48 | 0 | $NHSO_2OCH_2$- 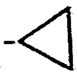 | H | $O-CH_3$ | |
| 49 | 0 | $NO_2$ | H | $N(CH_3)_2$ | |
| 50 | 0 | $NO_2$ | H | $S-CH_3$ | |
| 51 | 0 | $NO_2$ | H | $O-CH_3$ | Fp. 173°C |
| 52 | 0 | $O-CH_2$- 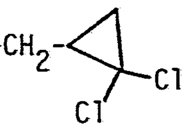 | H | $O-CH_3$ | |
| 53 | 0 | $O-CH_2$- 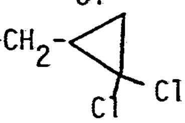 | H | $N(CH_3)_2$ | |
| 54 | 0 | $CH_2$- 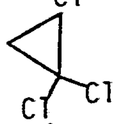 | H | $O-CH_3$ | |
| 55 | 0 | $CH_2$- 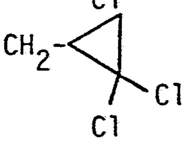 | H | $N(CH_3)_2$ | |
| 56 | 0 | $SO_2NH_2$ | H | $O-CH_3$ | |
| 57 | 0 | $SO_2NHCH_3$ | H | $O-CH_3$ | |
| 58 | 0 | $SO_2N(CH_3)_2$ | H | $O-CH_3$ | |
| 59 | 0 | $OSO_2N(CH_3)_2$ | H | $N(CH_3)_2$ | |
| 60 | 1 | $SO_2NH_2$ | H | $O-CH_3$ | |
| 61 | 1 | $SO_2N(CH_3)_2$ | H | $N(CH_3)_2$ | |
| 62 | 0 | Cl | H | Cl | Fp. 155°C |
| 63 | 0 | $COOCH_3$ | H | $CH_3S$ | Fp. 185°C |
| 64 | 0 | H | H | $OCH_3$ | |
| 65 | 0 | H | H | $NH_2$ | |

| Verb. No. | n | $R^1$ | $R^2$ | $R^3$ | physikal. Daten |
|---|---|---|---|---|---|
| 66 | 0 | H | H | $N(CH_3)_2$ | |
| 67 | 0 | H | H | $CH(CH_3)_2$ | |
| 68 | 0 | $COOCH_3$ | H | H | |
| 69 | 0 | $COOCH_3$ | H | OH | |
| 70 | 0 | $COOCH_3$ | H | SH | |
| 71 | 0 | $COOCH_3$ | H | $CH_3$ | |
| 72 | 0 | Cl | H | $CH_3$ | |

Beispiel 73:

2-Amino-4-methoxypyrido [2,3-d] pyrimidin

4,94 g 2,4-Diamino-6-methoxypyrimidin werden in 120 ml Eisessig zum Sieden erhitzt und nach Zusatz von 6,4 ml 1,1,3,3-Tetramethoxypropan noch 45 min. bei Siedetemperatur belassen. Anschließend wird der Eisessig abdestilliert und mehrfach mit Wasser koevaporiert und der Rückstand in 400 ml Wasser aufgenommen. Nach Zugabe von 5 g Natriumbicarbonat wird der vorhandene Niederschlag abgesaugt und verworfen.

Das Filtrat wird mehrfach mit Chloroform ausgeschüttelt, die vereinigten Extrakte mit Natriumbicarbonatlösung gewaschen und dann über Natriumsulfat getrocknet.

Der nach dem Einengen verbleibende Rückstand wird an Kieselgel mit Chloroform und einem Chloroform/Methanol-Gemisch gereinigt und danach aus Wasser umkristallisiert.

<u>Ausbeute:</u> 2,5 g (40,5 %) 2-Amino-4-methoxy-pyrido/2,3-d/-pyrimidin als Kristalle vom Schmp. 178-180°C.

| | | | | |
|---|---|---|---|---|
| Elementaranalyse: | ber. | C 54,54 | H 4,58 | N 31,80 |
| | gef. | C 54,42 | H 4,56 | N 31,77 |

## Beispiel 74:

### 2-Amino-4-chlor-pyrido [2,3-d] pyrimidin

Ausgehend von 2,6-Diamino-4-chlorpyrimidin und 1,1,3,3-Tetramethoxypropan wird analog Beispiel 71 nach chromatographischer Reinigung an Kieselgel mit Methanol/Diisopropylether (1:1) die Titelverbindung als gelbes Öl isoliert, welches ohne weitere Reinigung in den Folgereaktionen eingesetzt werden kann.

## Beispiel 75:

### 2-Amino-4-mercaptopyrido [2,3-d] pyrimidin

2-Amino-4-hydroxypyrido [2,3-d] pyrimidin (8,1 g) wird mit Phosphorpentasulfid (25 g) in Pyridin (125 ml) 10 h zum Sieden erhitzt. Nach Abdestillieren des Lösungsmittels wird der Rückstand mit Wasser verrieben, mit Natronlauge alkalisch gestellt, mit Aktivkohle gereinigt und das Filtrat mit Essigsäure angesäuert. Das ausfallende gelbe feinkristalliene Pulver wird mit Wasser gewaschen und getrocknet.

Man erhält 2-Amino-4-mercaptopyrido/2,3-d/pyrimidin-monohydrat (2,8 g; 32 %) als gelbes Pulver vom Schmp. 352°C.

Beispiel 76:

2-Amino-4-methylmercaptopyrido [2,3-d] pyrimidin

2-Amino-4-mercaptopyrido [2,3-d] pyrimidin (1,78 g; 10 mmol) werden in wäßriger Kalilauge (1,0 g KOH in 25 ml $H_2O$) gelöst und unter Rühren bei Raumtemperatur mit Methyljodid (1 ml) versetzt. Nach 30 min. Reaktionszeit wird die ausfallene Substanz abgesaugt, mit wenig Ethanol verrieben und getrocknet.

Man erhält 2-Amino-4-methylmercaptopyrido[2,3-d]pyrimidin-monohydrat(1,8 g; 86 %) als gelbes Pulver vom Schmp. 180°C.

Beispiel 77:

2-Amino-4-dimethylamino-pyrido [2,3-d] pyrimidin

2-Amino-4-methylmercaptopyrido [2,3-d] pyrimidin (1,05 g; 5 mmol) werden in einer alkoholischen Dimethylaminlösung (30 ml; 30 % Dimethylamin in Ethanol) 4 Stunden bei 80°C belassen. Nach Abdestillieren des Lösungsmittels wird der Rückstand mit Diethylether verrieben und von dem Produkt abgesaugt.

Man erhält in nahezu quantitativer Ausbeute 2-Amino-4-dimethylaminopyrido [2,3-d] pyrimidin (1,0 g; 99 %) vom Schmp. 200°C.

Herbizide Wirkung in Vorauflaufverfahren

Die Pflanzen werden 2 cm tief in Töpfe eingesät und am Tag der Einsaat mit einem Spritzbrühenaufwand von 600 bzw. 800 l/ha mit einer Bandspritzanlage auf die Oberfläche des abdeckenden Bodens gespritzt und im Gewächshaus aufgestellt. Die Wirkung wird nach 3 Wochen im Vergleich zur unbehandelten Kontrolle bestimmt und im Prozent angegeben (0 % = ungeschädigt bis 100 %= vollständig abgestorben).
Die Verbindungen der Formel I zeigen gute bis sehr gute Wirkung.

Hervorzuheben ist Verb. No. 6 (Tab. 1), deren Wirkung bei verschiedenen Unkräutern und Ungräsern bei einer Aufwandmenge von 0,1 kg/ha wie folgt ist:

| Art | Schädigung/Absterben % |
|---|---|
| | 0,1 kg/ha |
| Echinochloa crus-galli | 86 |
| Alopecurus myosuroides | 92 |
| Avena fatua | 70 |
| Setaria viridis | 98 |
| Digitaria sanguinalis | 87 |
| Solanum nigrum | 82 |
| Sinapis alba | 95 |
| Lamium amplexicaule | 98 |
| Centaurea cyanus | 88 |
| Stellaria media | 96 |
| Veronica persicaria | 99 |
| Galium aparine | 91 |
| Matricaria inodora | 97 |

Als Stand der Technik wird mit dem handelsüblichen Sulfonylharnstoff Chlorsulfuron (N-2-Chlorsulfonyl-N'-(4-methoxy-6-methyl-1,3,5-triazin-2-yl)harnstoff) bei Cyperus esulentus verglichen.

| Aufwandmenge | Bsp. 6 Tab. 1 | Chlorsulfuron |
|---|---|---|
| 0,2 kg/ha | 99 % | 41 |
| 0,1 kg/ha | 97 % | 0 |

Herbizide Wirkung im Nachauflaufverfahren

Die Pflanzen werden 2 cm tief in Töpfe eingesät und bis zum 2,5-Blattstadium (Monocotyle/Granineen) bzw. bis zum 3-4-Blattstadium (Cyperus esculentus) bzw. bis zum 1,5 Folgeblattstadium vorkultiviert und dann mit einer Bandspritzanlage mit einem Spritzbrühenaufwand von 600 bzw. 800 l/ha auf die Blätter gespritzt und im Gewächshaus aufgestellt.

Die Wirkung wird nach 3 Wochen im Vergleich zur unbehandelten Kontrolle bestimmt und in Prozent angegeben (0 % = ungeschädigt bis 100 % = vollständig abgestorben).

Die Verbindungen der Formel I zeigen gute bis sehr gute Wirkung.

Hervorzuheben ist Verbindung No. 6 (Tab. 1), deren Wirkung bei verschiedenen Unkräutern und Ungräsern bei einer Aufwandmenge von 0,1 kg/ha wie folgt ist:

| Art | Schädigung/Absterben, % |
|---|---|
| | 0,1 kg/ha |
| Echinochloa crus-galli | 93 |
| Alopecurus myosuroides | 94 |
| Avena fatua | 61 |
| Solanum nigrum | 99 |
| Sinapis alba | 99 |
| Lamium amplexicaule | 99 |
| Centaurea cyanus | 80 |
| Stellaria media | 99 |
| Veronica persicaria | 100 |
| Galuim aparine | 98 |
| Matricaria inodora | 97 |

Als Stand der Technik wird mit dem handelsüblichen Sulfonylharnstoff Chlorsulfuron (N-2-Chlorsulfonyl-N'-(4-methoxy-6-methyl-1,3,5-triazin-2-yl)harnstoff) bei Cyperus esculentus verglichen.

| Aufwandmenge | Bsp. 6 Tab. 1 | Chlorsulfuron |
|---|---|---|
| 0,2 kg/ha | 94 % | 0 % |
| 0,1 kg/ha | 90 % | 0 % |

Wachstumsregulierende Wirkung

Die wachstumsregulierende Wirkung wird bei behandeltem Saatgut und nach Blattspritzung junger Pflanzen bestimmt.

Saatgutbehandlung

Das Saatgut von Weizen und Gerste wird für 3 Stunden in Lösungen der Prüfsubstanz eingequollen, danach in dest. Wasser gespült und in feuchtem Vermiculit im Dunkeln inkubiert. Nach 12 Tagen wird die Gesamtlänge der etiolierten Keimpflanze gemessen.

Blattspritzung

Die Blattspritzung wird analog der Nachauflaufbehandlung durchgeführt.

Die Tabelle gibt die gemessene Länge in % im Vergleich zu Kontrollpflanzen (100 %) an.

| Substanz | Pflanzenlängen, % (Saatgutbehandlung) | | Pflanzenlängen, % (Blattspritzung) | | |
|---|---|---|---|---|---|
| Verb. No. | Weizen | Gerste | Weizen | Gerste | Reis |
| Einquellen 1000/10 ppm | 47/83 | 42/82 | --- | --- | --- |
| Spritzen 100 g/ha | --- | --- | 74 | 68 | 61 |
| Chlorsulfuron Einquellen 1000/10 ppm | 94/100 | 101/102 | --- | --- | --- |
| Spritzen 100 g/ha | --- | --- | 99 | 94 | --- |

Patentansprüche

1) Verbindungen der Formel

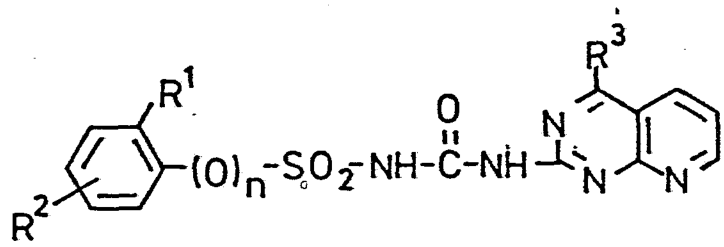

(I)

in der

n 0 oder 1

$R^1$,$R^2$ die gleich oder verschieden sein können Wasserstoff, Halogen, Cyano, Nitro, Niederalkoxycarbonyl, gegebenenfalls halogensubstituiertes Niederalkyl, gegebenenfalls halogensubstituiertes Niederalkyloxy, gegebenenfalls halogensubstituiertes Niederalkylthio, gegebenenfalls halogensubstituiertes Niederalkenyl, gegebenenfalls halogensubstituiertes Niederalkenyloxy, gegebenenfalls halogensubstituiertes Niederalkenylthio, gegebenenfalls niederalkylsubstituierte Cycloalkyl, Di-(niederalkyl)-amino, gegebenenfalls halogensubstituiertes Cyclopropylmethyl, gegebenenfalls halogensubstituiertes Cyclopropylmethyloxy, X $SO_2R^4$

$R^3$ Wasserstoff, gegebenenfalls halogensubstituiertes Niederalkyl, gegebenenfalls halogensubstituiertes Niederalkyloxy, gegebenenfalls halogensubstituiertes Niederalkylthio, Halogen, Amino, Hydroxy, Mercapto, Mono(niederalkyl)amino, Di-(niederalkyl)amino.

$R^4$ Niederalkyl, gegebenenfalls ein- bis dreifach halogensubstituiertes Niederalkyl oder Niederalkyloxy, Cyclopropyl, Methyl, Cyclopropylmethoxy, Amino, Mono(niederalkyl)amino, Di(niederalkyl)amino

X Sauerstoff, NH oder N(Niederalkyl) oder direkte C-S-Bindung

bedeuten,
in freier Form und als Salze mit Säuren und Basen.

2) Verbindungen der Formel (I) nach Anspruch 1, in der

n 0,1

$R^1$ Wasserstoff, Nitro, Fluor, Chlor, Niederalkyloxycarbonyl, gegebenenfalls halogensubstiuiertes Niederalkyl, gegebenenhalogensubstituiertes Niederalkyloxy, gegebenenfalls halogensubstituiertes Niederalkylthio, gegebenenfalls halogensubstituiertes Niederalkenyl, gegebenenfalls halogensubstituiertes Niederalkenyloxy, gegebenenfalls halogensubstituiertes Niederalkenylthio, gegebenenfalls halogensubstituiertes Cyclopropylmethyl, gegebenenfalls halogensubstituiertes Cyclopropylmethyloxy, Di-(nieder-alkyl)amino, X $SO_2R^4$

$R^2$ Wasserstoff, Chlor, Fluor

$R^3$ Wasserstoff, Fluor, Chlor, Niederalkyl, Niederalkyloxy, Niederalkylthio, Hydroxy, Mercapto, Amino, Mono(niederalkyl)amino, Di-(niederalkyl)amino, Trifluormethyl

$R^4$ Niederalkyl, Niederalkyloxy, Cyclopropylmethoxy, Amino, Di-(niederalkyl)amino,

X Sauerstoff, NH, N(Niederalkyl) oder direkte C-S-Bindung

bedeuten.

3) Verbindungen der Formel (I) nach Anspruch 1, in der

n 0

$R^1$ Methoxycarbonyl, Chlor, Nitro

$R^2$ Wasserstoff

$R^3$ Amino, Methoxy, Dimethylamino, Methylthio, Chlor, Hydroxy, Mercapto, Methylamino

bedeuten

4) N-(2-Methoxycarbonylphenylsulfonyl)-N'-(4-methoxypyrido [2,3-d] pyrimidin-2-yl)harnstoff.

5) Herbizides Mittel gekennzeichnet durch einen Gehalt an einer Verbindung der Formel (I).

6) Verfahren zur Bekämpfung von Unkräutern, dadurch gekennzeichnet, daß man Verbindungen der Formel (I) auf die Unkräutern und/ oder deren Lebensraum ausbringt.

7) Wachstumsregulierendes Mittel gekennzeichnet durch einen Gehalt einer Verbindung der Formel (I).

8) Verfahren zur Beeinflussung des Längenwachstums von Kulturpflanzen, dadurch gekennzeichnet, daß man Verbindungen der Formel (I) auf die Kulturpflanzen, deren Samen oder deren Lebensraum einwirken läßt.

9) Verfahren zur Herstellung von Verbindungen nach Anspruch 1 bis 11
dadurch gekennzeichnet, daß man

a) ein Isocyanat der Formel

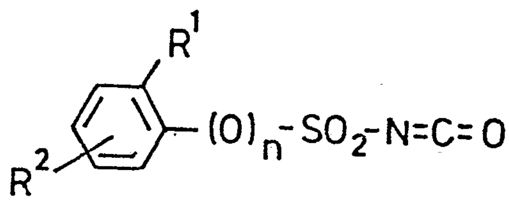

(II)

in dem n, $R^1$ und $R^2$ die obige Bedeutung haben, mit einem Amin der Formel

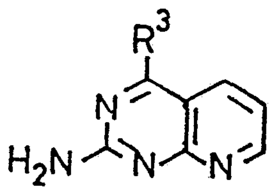

(III)

in der $R^3$ die obige Bedeutung hat, umsetzt, oder daß man

b) ein Carbamat der Formel

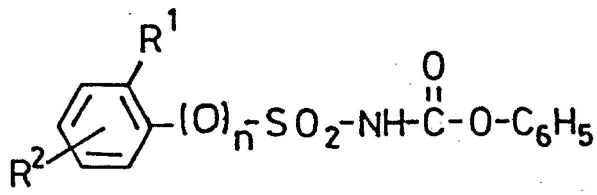

(IV)

in dem n, $R^1$ und $R^2$ die obige Bedeutung haben mit einem Amin der Formel (III) umsetzt, oder daß man

c) eine Verbindung der Formel

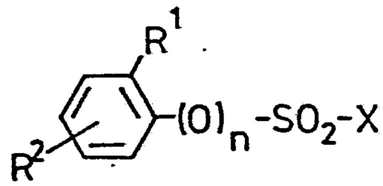

(V)

in der n, $R^1$ und $R^2$ die obige Bedeutung haben und X für Halogen, vorzugsweise Chlor steht, mit einem Imidokohlensäureester der Formel

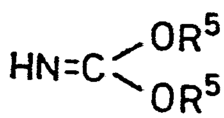

(VI)

in der $R^5$ für einen Niederalkyl-, Phenyl- oder Benzylrest steht zu einem Imidokohlensäureester der Formel

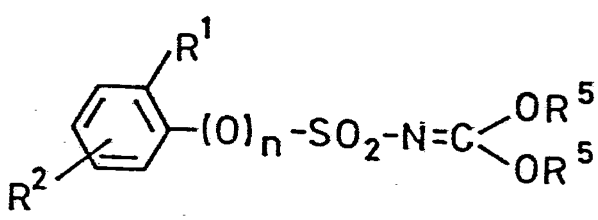

(VII)

umsetzt und danach mit einem Amin der Formel (III) umsetzt und den so erhaltenen Isoharnstoff der Formel

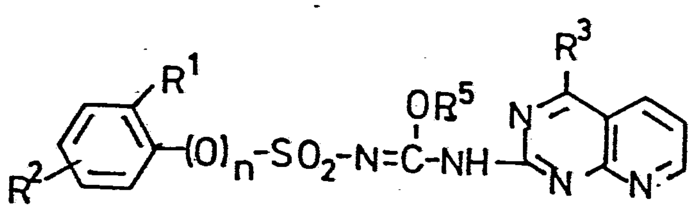

(VIII)

mit einer Halogenwasserstoffsäure zu einer Harnstoffverbindung der Formel (I) spaltet,

d) eine Verbindung der Formel

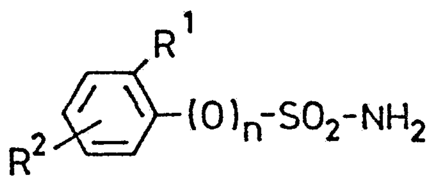

(IX)

in der n, $R^1$ und $R^2$ die obige Bedeutung haben, zusammen mit Carbodiimidazol und einem Amin der Formel (III) umsetzt und gewünschtenfalls die nach a), b), c) oder d) erhaltenen Verbindungen der Formel (I) in üblicher Weise in Salze überführt.

10) 2-Aminopyrido [2,3-d] pyrimidine der Formel

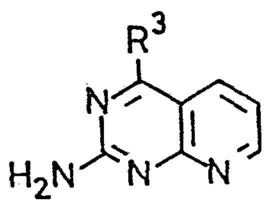

(III)

worin $R^3$ wie zuvor definiert ist, aber nicht für Hydroxy und Amino steht.

11) Verfahren zur Synthese von Verbindungen der Formel

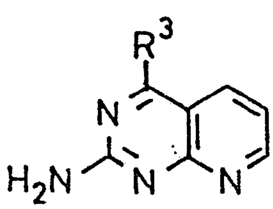

(III)

worin $R^3$ wie zuvor definiert ist, dadurch gekennzeichnet, daß ein Pyrimidin der Formel

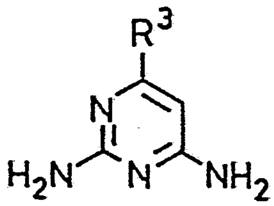

(X)

mit Malondialdehyd oder einem funktionellen Derivat des Malondialdehydes umgesetzt wird.